# EUROPEAN PATENT SPECIFICATION

(11) **EP 2 416 735 B1**
(45) Date of publication and mention of the grant of the patent: **17.07.2013**
(21) Application number: 10715000.5
(22) Date of filing: 06.04.2010
(51) Int. Cl.: A61F 2/08

(54) **TISSUE GRAFT ANCHOR**
GEWEBETRANSPLANTATANKER
ANCRAGE DE GREFFE DE TISSU

(30) Priority: 06.04.2009 US 166908 P
(43) Date of publication of application: 15.02.2012
(73) Proprietor: Smith & Nephew, Inc., Memphis, TN 38116 (US)
(72) Inventor: MONTES, Horacio, De Oca, York YO26 5LR (GB); BROWN, Malcolm, Otley (GB); HALL, Michael, Linthorpe, Middlesbrough (GB); HOWLING, Graeme, Roundhay, Leeds (GB)
(74) Representative: Guy, Mark Robert
(86) International application number: PCT/US2010/030043
(87) International publication number: WO 2010/117982

(56) References cited:
- WO-A2-03/088818
- WO-A2-2006/108114
- WO-A2-2008/130954

## Description

### BACKGROUND

### FIELD OF TECHNOLOGY

The present disclosure relates to tissue graft fixation.

### RELATED ART

A ligament, such as an anterior cruciate ligament (ACL), that has ruptured and is non-repairable, is generally replaced arthroscopically by a tissue graft. The tissue graft can be harvested from a portion of a patellar tendon having so called "bone blocks" at each end, and from the semitendonosis and gracilis. Alternatively, the tissue graft can be formed from synthetic materials or from a combination of synthetic and natural materials.

The replacement tissue graft is implanted by securing one end of the tissue graft in a socket formed in a passage within the femur, and passing the other end of the graft through a passage formed in the tibia. Generally, sutures are used to affix each end of the tissue graft to an anchor (e.g., an interference screw or a post), which is then secured to the bone. However, disadvantages of a traditional interference screw include the requirement of a range of screw sizes for different patients, the lack of 360° contact of the screw with the treatment area, tendon slippage, and a low fixation force by the screw on the tendon.
WO06108114 describes a device for use as a bone implant. The device includes a body having a pre-implantation shape and a post-implantation shape different from the pre-implantation shape. The body is configured to change from the pre-implantation shape to the post-implantation shape in response to the body being activated. The body is configured to be inserted in a bone recess while the body is in the pre-implantation shape.

There remains a need for a tissue graft anchor which is simple, easy to install, and inexpensive to manufacture, while providing secure, trouble-free anchoring of a tissue graft.

### SUMMARY

In an aspect, the present disclosure relates to a method of fixating a tissue graft. The method includes creating a hole in bone; inserting a tissue graft into the hole; inserting a cannulated anchor into the hole, the anchor including threads on an outer surface of the anchor and a polymer material including shape memory qualities; and providing energy to the anchor to deform the anchor and fixate the anchor to the bone, wherein upon deformation of the anchor, a depth of each thread of the anchor is substantially reduced thereby increasing contact between the anchor and the tissue graft.

In an embodiment, the bone includes a femur. In another embodiment, the bone includes a tibia. In yet another embodiment, the polymer material is selected from a group including an amorphous polymer, a semi-crystalline polymer, and combinations thereof. In a further embodiment, the polymer material includes a resorbable polymer material. In yet a further embodiment, the polymer material includes a non-resorbable polymer material. In an embodiment, a pitch of each thread of the anchor is substantially reduced. In another embodiment, the source of energy includes body temperature.

In another aspect, the present disclosure relates to an anchor. The anchor includes a proximal portion; a distal portion having threads; a middle portion located between the proximal portion and the distal portion, the middle portion including a diameter that is smaller than a diameter of the proximal portion and the distal portion; and a polymer material coupled to the middle portion, the polymer material having shape memory qualities.

In yet another aspect, the present disclosure relates to an anchor. The anchor includes threads on an outer surface of the anchor and a polymer material including shape memory qualities, wherein upon providing the anchor with energy, the anchor deforms such that a depth of each thread of the anchor is substantially reduced. In an embodiment, a pitch of each thread
of the anchor is reduced. In another embodiment, the source of energy includes body temperature.

In a further aspect, the present disclosure relates to an anchor. The anchor includes a proximal portion, a distal portion, a cannulation, and an outer surface including a plurality of barbs and a plurality of grooves, the grooves extending an entire length of the anchor, wherein the anchor includes a polymer material having shape memory qualities.

Further areas of applicability of the present disclosure will become apparent from the detailed description provided hereinafter. It should be understood that the detailed description and specific examples, while indicating the preferred embodiment of the disclosure, are intended for purposes of illustration only and are not intended to limit the scope of the disclosure.

### BRIEF DESCRIPTION OF THE DRAWINGS

The accompanying drawings, which are incorporated in and form a part of the specification, illustrate the embodiments of the present disclosure and together with the written description serve to explain the principles, characteristics, and features of the disclosure. In the drawings:

Fig. 1 shows a flow chart of the method of the present disclosure.

Figs. 2A-2B illustrate an embodiment of the method of Fig. 1.

Fig. 3 shows a cross-sectional view of a first embodiment of a tissue graft anchor of the present disclosure.

Figs. 4A-4B show a second embodiment of a tissue graft anchor of the present disclosure.

Figs. 5A-5B show the tissue graft anchor of Figs. 4A-4B after insertion of the anchor into bone and providing the anchor with energy.

### DETAILED DESCRIPTION OF THE EMBODIMENTS

The following description of the preferred embodiment(s) is merely exemplary in nature and is in no way intended to limit the disclosure, its application, or uses.

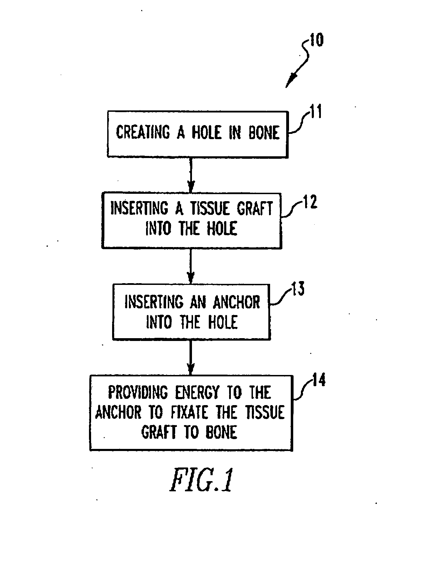

Fig. 1 shows a flow chart of the method **10** of the present disclosure. The method **10** includes creating a hole in bone **11;** inserting a tissue graft into the hole **12;** inserting an anchor into the hole **13;** and providing energy to the anchor to deform the anchor and fixate the tissue graft to the bone **14.**

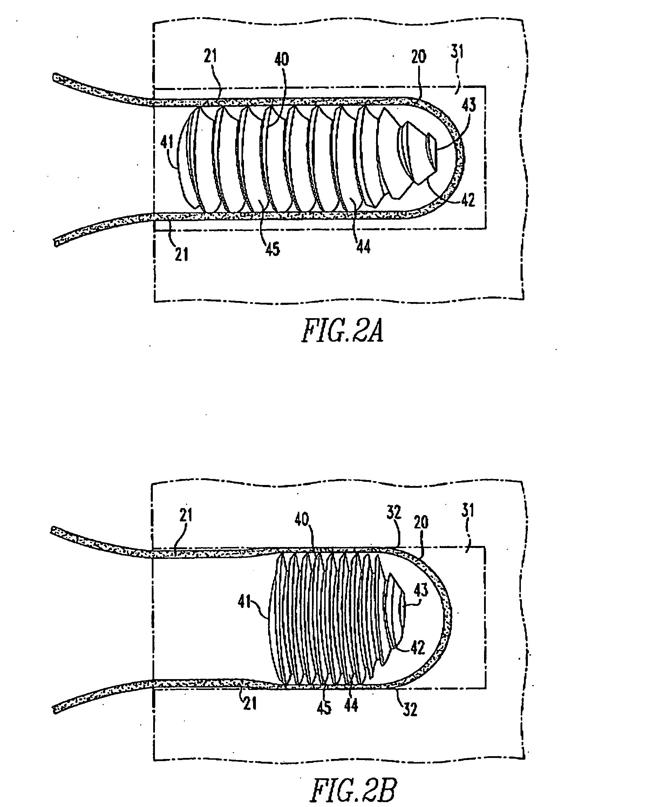

An example of this method **10** is shown in Figs. 2A-2B. Fig. 2A shows ends **21** of a tissue graft **20** housed within a bone tunnel 31 and an anchor **40** disposed between the ends **21.** Creation of the bone tunnel **31** and insertion of the tissue graft **20** into the bone tunnel **31** may be done via any method known to one of skill in the art. The anchor **40** includes a proximal end **41** and a distal end **42.** In addition, the anchor **40** is tapered from the proximal end **41** to the distal end **42.** A cannulation **43** exists along the entire length of the anchor **40.** However, an anchor having a cannulation extending a partial length of the anchor is also within the scope of this disclosure. Furthermore, the anchor **40** includes an outer surface **44** having threads **45.** The anchor **40** is inserted into the bone tunnel **31,** via a rotary motion, using any instrument known to one of skill in the art for inserting an anchor into bone.

For the purposes of this disclosure, the anchor **40** includes a resorbable polymer material having shape memory qualities. Specific polymers that may be used include polyetherctherketone (PEEK), polymethyl methacrylate (PMMA), polyethyl methacrylate (PEMA), polyacrylate, poly-alpha-hydroxy acids, polycapropactones, polydioxanones, polyesters, polyglycolic acid, polyglycols, polylactidcs, polyorthoesters, polyphosphates, polyoxaesters, polyphosphoesters, polyphosphonates, polysaccharides, polytyrosine carbonates, polyurethanes, polyhydroxyethylmethacrylate (PHEMA), polyethylene glycol (PEG), trimethyl carbonate, and copolymers or polymer blends thereof. In addition, bioactive agents may be incorporated into the polymer material to be released during the deformation or the degradation of the polymer material. These agents are included to help promote bone regrowth. Examples include bone morphogenic proteins, antibiotics, anti-inflamatoies, angiogenic factors, osteogenic factors, monobutyrin, omental extracts, thrombin, modified proteins, platelet rich plasma/solution, platelet poor plasma/solution, bone marrow aspirate, and any cells sourced from flora or fawna, such as living cells, preserved cells, dormant cells, and dead cells. Other bioactive agents known to one of ordinary skill in the art may also be used. Furthermore, the polymeric materials can be formed as a composite or matrix and include reinforcing material or phases such as fibers, rods, platelets, and fillers. For example, the polymeric material can include glass fibers, carbon fibers, polymeric fibers, ceramic fibers, or ceramic particulates. Other reinforcing material or phases known to one of ordinary skill in the art could also be used.

Furthermore, one or more hydrophilic materials may be included in the polymer matrix to accelerate water ingress and hence the relaxation rate of the polymer material. Examples of hydrophilic materials include, as stated above, polyethylene glycol. Other hydrophilic materials known to one of ordinary skill in the art may also be used.

The oriented polymer material may also include a porogen, such as sodium chloride. Other porogens known to one of ordinary skill in the art may also be used. The porogen may then be washed out of the material leaving pores that will aid water penetration and hence accelerate the relaxation rate of the material. Porogens may be included in the polymer material and washed out to leave pores before the material is oriented. Upon orientation of the material, channels will develop in the material, due to an increase in surface area, to aid in water penetration and relaxation rate. Since the rate of relaxation is dependent upon the diffusion rate of fluid into the polymer, the addition of these channels, pores, porogens, and hydrophilic units enhances the rate of relaxation of these materials.

Factors used to determine the type of polymer used include, but are not limited to, the desired amount of polymer deformation, the desired rate at which that deformation occurs, the rate at which the polymer is absorbed, and the strength of the polymer.

Generally, polymers that display shape memory qualities show a large change in modulus of elasticity at the glass transition temperature (T_{g}). The shape-memory function can be achieved by taking advantage of this characteristic. Namely, a polymer material having a first shape (original shape) is processed to produce an article with a second shape (defined shape). This process also provides the material with its shape memory qualities by imparting an orientation to the polymer chains of the material. The article is then provided with energy to heat the article to a temperature (T_{f}) higher than the Tg of the polymer, but lower than the melting temperature (Tₘ) thereof so as to deform the article and return it to its original shape.

The anchor **40** shown in Fig. 2A represents the article described above having the defined shape. Once the anchor **40** has been provided with energy, it is deformed, as shown in Fig. 2B, to return the anchor **40** to its original shape. Upon deformation, the anchor **40** changes shape by shrinking axially, or along the length of the material, and expanding radially, or along the width of the material. This expansion and shrinkage causes the anchor **40** to engage the ends **21** of the tissue graft **20** and force the ends **21** towards the wall **32** of the bone tunnel **31**, thereby fixating the tissue **20** to the bone **30.** In addition to this shrinkage and expansion, upon deformation, a depth and pitch of each thread of the anchor **40** is substantially reduced thereby increasing contact between the anchor and the tissue graft.

An energy source that may be used to deform the anchor **40** includes a heated liquid, such as saline or water. The liquid may be introduced into the bone tunnel **31** via a syringe or other delivery device. In addition, an electrosurgical heating probe, such as the electrosurgical heating probes described in International Patent Application Publication WO 2008/112880, may also be used. It is also within the scope of this disclosure that once the anchor 40 is placed in the bone, body heat would be transferred from blood and tissue, via thermal conduction, to provide the energy necessary to deform the anchor **40.** In this instance, body temperature would be used as a thermal energy source.

The anchor 40 may be formed by die-drawing, extrusion, cold forging, or molding (i.e. compression flow molding or thermoforming process) the above-mentioned polymers or polymer compositions. Other solid-state processing methods may also be used.

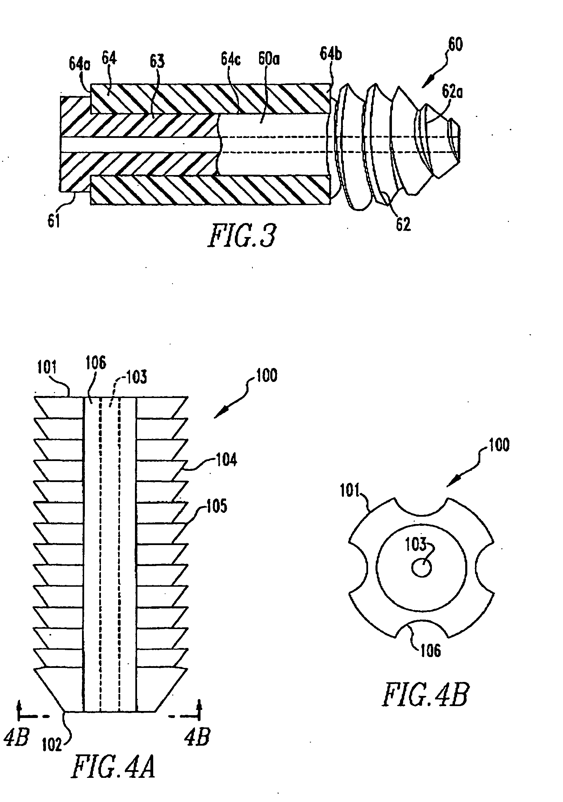

Fig. 3 shows a cross-sectional view of an anchor **60** including a body **60a** having a proximal portion **61,** a distal portion **62** having threads **62a,** and **a** middle portion **63** located between the proximal portion **61** and the distal portion **62.** The middle portion **63** includes a diameter that is smaller than a diameter of the proximal portion **61** and the distal portion **62.** A sleeve **64** of polymer material is coupled to the middle portion **63.** The sleeve **64** includes a proximal end **64a,** a distal end **64b,** and a cannulation **64c.** The polymer material includes shape memory qualities. The anchor **60** may be used in coupling tissue to bone by inserting the anchor **60** into **a** bone tunnel and providing the anchor **60** and, specifically the sleeve **64,** with energy to expand the sleeve **64** and fixate the tissue to bone, similar to what has been described above. During insertion of the anchor **60** into the bone, the body **60a** of the anchor **60** rotates and the sleeve 64 remains static, thereby allowing the tissue to remain in alignment with the anchor **60.**

Figs. 4A-4B show an anchor **100** including a proximal portion **101,** a distal portion **102,** a cannulation **103,** and an outer surface **104** including barbs **105** and grooves **106.** The grooves **106** extend an entire length of the anchor **100** and are more clearly shown in Fig. 4B. The anchor **100** includes a polymer material having shape memory qualities.

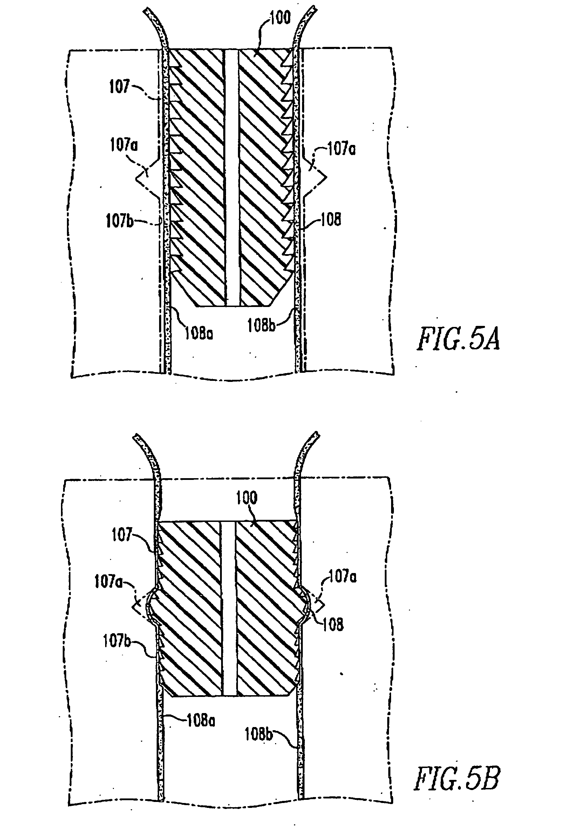

As shown in Figs. 5A and 5B, the anchor **100** is inserted into a prepared bone tunnel 107, subsequent to insertion of soft tissue **108** into the bone tunnel **107,** such that the ends **108a,108b** of the tissue **108** are housed within the grooves **106.** The tunnel **107** includes an undercut portion **107a,** as shown more clearly in Fig. 5A, for purposes to be further described below. Once the anchor **100** has been inserted into the tunnel **107,** the anchor **100** is provided with energy, via a method described above, to deform the anchor **100**, thereby causing the anchor **100** to engage the ends **108a,108b** of the soft tissue **108** and force the ends **108a,108b** towards the wall **107b** of the tunnel **107,** as shown more clearly in Fig. **5B**. Also shown in Fig. 5B is the expansion of the anchor **100** into the undercut portions **107a**. It is believed that expansion of the anchor **100** and, therefore the ends **108a,108b,** into the undercut portions **107a** will serve to lock the ends **108a,108b** within the portion **107a,** thereby further increasing fixation of the tissue **108** within the bone tunnel **107.** Furthermore, the barbs **105** provide for more secure engagement of the soft tissue **108** and, therefore, increased fixation of the soft tissue **108** to the bone tunnel **107.**

### EXAMPLE ONE

A polymer rod, having shape memory qualities and a diameter of about 15 mm, was produced by die drawing a 30 mm diameter billet of Poly(D,L-lactide-co-glycolide) 85:15 containing 35% ^{w}/w NaCl through a 15 mm die. The polymer rod was machined to produce several samples, each of which had a diameter about 13 mm and a length of about 35 mm. A mold with two symmetrical halves and a threaded hole of length 41.35 mm and diameter 13.78 mm (narrowest part of thread) was clamped shut and heated to 80°C in an oven. A machined sample was placed in the threaded hole of the mold and heated in the oven for 2.5 minutes. The mold was then removed from the oven and crash cooled by immersion in liquid N₂. Once the mold had cooled it was removed from the liquid N₂ and opened. The sample had shape recovered to take the shape of the threaded hole in the mold. The molded sample was cut in half and one half was placed in hot water at approx. 90°C and removed once the shape recovery process had stopped. Once the sample was dry, its diameter was measured as 22.7 mm, having recovered to 87% of the theoretical recovery diameter. The pitch and the depth of the threads introduced during the molding process had also been substantially reduced.

### EXAMPLE TWO

Two pieces of rope, each having the same length and representing soft tissue, were inserted into a hole in sawbone, such that the ends of the ropes both extended from the sawbone. A molded sample from example one was placed within the hole of the sawbone, such that the ropes were located between the sample and the inner wall of the hole. The sample was provided with energy, via inserting hot water, having a temperature of 80° C, into the hole, which caused the sample to deform and force the pieces of rope against the inner wall of the hole, thereby fixating the ropes within the hole. The ropes were removed from the hole by pulling on the rope ends and the pull-out force was tested. The pull-out force for the deformed sample was 1000 N.

As a control measure, a second molded sample was inserted into a hole of a second sawrbone in the same manner as described above, without providing the sample with energy. The ropes were removed from the hole by pulling on the rope ends and the pull-out force was tested. The pull-out force for the second sample was 500 N.

Therefore, the deformed sample did provide for substantial fixation of the ropes to the bone as compared to the control sample, which was reflected in the amount of increase in pull-out force between the two samples.

As various modifications could be made to the exemplary embodiments, as described above with reference to the corresponding illustrations, without departing from the scope of the disclosure, it is intended that all matter contained in the foregoing description and shown in the accompanying drawings shall be interpreted as illustrative rather than limiting. Thus, the breadth and scope of the present disclosure should not be limited by any of the above-described exemplary embodiments, but should be defined only in accordance with the following claims appended hereto and their equivalents.

## Claims

1. An anchor (60) comprising;
a proximal portion (61);
a distal portion (62) having threads (62a);
a middle portion (63) located between the proximal portion and the distal portion, the middle portion including a diameter that is smaller than a diameter of the proximal portion and the distal portion; and **characterised in**
a polymer material coupled to the middle portion, the polymer material having shape memory qualities.

2. The anchor of claim 1 further comprising:
threads on an outer surface of the anchor and a polymer material including shape memory qualities,
wherein upon providing the anchor with energy, the anchor deforms such that a depth of each thread of the anchor is substantially reduced.

3. The anchor of claim 1 or claim 2 wherein the polymer material is selected from a group consisting essentially of an amorphous polymer, a semi-crystalline polymer, and combinations thereof.

4. The anchor of claim 1 or claim 2 wherein the polymer material comprises a resorbable polymer material.

5. The anchor of claim 1 or claim 2 wherein the polymer material comprises a non-resorbable polymer material.

6. The anchor of claim 2 wherein a pitch of each thread of the anchor is substantially reduced.

7. The anchor of claim 2 wherein the source of energy comprises body temperature.

8. The anchor of claim 1 wherein the polymer material is in the form of a sleeve (64).

9. The anchor of claim 8, wherein the anchor is freely rotatable relative to the sleeve.

## Patentansprüche

1. Ein Anker (60), der Folgendes beinhaltet:
einen proximalen Abschnitt (61);
einen distalen Abschnitt (62), der Gewinde (62a) aufweist;
einen mittleren Abschnitt (63), der zwischen dem proximalen Abschnitt und dem distalen Abschnitt gelegen ist, wobei der mittlere Abschnitt einen Durchmesser umfasst,
der kleiner als ein Durchmesser des proximalen Abschnitts und des distalen Abschnitts ist; und **gekennzeichnet durch**
ein Polymermaterial, das an den mittleren Abschnitt gekoppelt ist, wobei das Polymermaterial Formgedächtniseigenschaften aufweist.

2. Anker gemäß Anspruch 1, der ferner Folgendes beinhaltet:
Gewinde auf einer äußeren Oberfläche des Ankers und ein Polymermaterial, das Formgedächtniseigenschaften umfasst,
wobei, wenn der Anker mit Energie versorgt wird, sich der Anker verformt, so dass eine Tiefe jedes Gewindes des Ankers im Wesentlichen reduziert wird.

3. Anker gemäß Anspruch 1 oder Anspruch 2, wobei das Polymermaterial aus einer Gruppe, die grundsätzlich aus einem amorphen Polymer, einem teilkristallinen Polymer und Kombinationen daraus besteht, ausgewählt ist.

4. Anker gemäß Anspruch 1 oder Anspruch 2, wobei das Polymermaterial ein resorbierbares Polymermaterial beinhaltet.

5. Anker gemäß Anspruch 1 oder Anspruch 2, wobei das Polymermaterial ein nicht resorbierbares Polymermaterial beinhaltet.

6. Anker gemäß Anspruch 2, wobei ein Abstand jedes Gewindes des Ankers im Wesentlichen reduziert wird.

7. Anker gemäß Anspruch 2, wobei die Energiequelle Körpertemperatur beinhaltet.

8. Anker gemäß Anspruch 1, wobei das Polymermaterial in der Form einer Hülse (64) vorliegt.

9. Anker gemäß Anspruch 8, wobei der Anker relativ zu der Hülse frei drehbar ist.

## Revendications

1. Une pièce d'ancrage (60) comprenant :
une portion proximale (61) ;
une portion distale (62) présentant un filetage (62a) ;
une portion de milieu (63) située entre la portion proximale et la portion distale, la portion de milieu ayant un diamètre qui est plus petit qu'un diamètre de la portion proximale et de la portion distale ; et caractérisée en
un matériau polymère couplé à la portion de milieu, le matériau polymère présentant des qualités de mémoire de forme.

2. La pièce d'ancrage de la revendication 1 comprenant de plus :
un filetage sur une surface externe de la pièce d'ancrage et un matériau polymère ayant des qualités de mémoire de forme,
la pièce d'ancrage se déformant, lorsque de l'énergie lui est apportée, de sorte qu'une profondeur de chaque filet de la pièce d'ancrage est substantiellement réduite.

3. La pièce d'ancrage de la revendication 1 ou de la revendication 2 dans laquelle le matériau polymère est choisi dans un groupe consistant essentiellement en un polymère amorphe, un polymère semi-cristallin et des combinaisons de ceux-ci.

4. La pièce d'ancrage de la revendication 1 ou la revendication 2 dans laquelle le matériau polymère comprend un matériau polymère résorbable.

5. La pièce d'ancrage de la revendication 1 ou la revendication 2 dans laquelle le matériau polymère comprend un matériau polymère non résorbable.

6. La pièce d'ancrage de la revendication 2 dans laquelle un pas de chaque filet de la pièce d'ancrage est substantiellement réduit.

7. La pièce d'ancrage de la revendication 2 dans laquelle la source d'énergie comprend la température corporelle.

8. La pièce d'ancrage de la revendication 1 dans laquelle le matériau polymère se présente sous la forme d'un manchon (64).

9. La pièce d'ancrage de la revendication 8, la pièce d'ancrage pouvant tourner librement relativement au manchon.
